# EUROPÄISCHE PATENTSCHRIFT

(11) **EP 1 938 757 B1**
(45) Veröffentlichungstag und Bekanntmachung des Hinweises auf die Patenterteilung: **14.07.2010**
(21) Anmeldenummer: 07024667.3
(22) Anmeldetag: 19.12.2007
(51) Int. Cl.: A61B 17/00, A61C 1/14, A61B 17/16, A61B 17/22, A61B 17/32

(54) **Chirurgische Kupplungsvorrichtung**
Surgical coupling device
Dispositif de couplage chirurgical

(30) Priorität: 27.12.2006 DE 102006062421
(43) Veröffentlichungstag der Anmeldung: 02.07.2008
(73) Patentinhaber: Gebr. Brasseler GmbH & Co. KG, 32657 Lemgo (DE)
(72) Erfinder: Küllmer, Michael, 32657 Lemgo (DE); Teller, Martin, 32657 Lemgo (DE)
(74) Vertreter: Weber, Joachim

(56) Entgegenhaltungen:
- US-A- 5 871 493

## Beschreibung

Die Erfindung bezieht sich auf eine chirurgische Kupplungsvorrichtung zur lösbaren Verbindung eines Handstücks mit einem chirurgischen Instrument.

Chirurgische Instrumente, welche in ein Handstück eingesetzt werden, sind in unterschiedlichsten Ausgestaltungsformen bekannt. Die Handstücke sind üblicherweise mit einem Antrieb verbunden, so dass Werkzeuge oder Teilbereiche der chirurgischen Instrumente antreibbar, beispielsweise in Drehung versetzbar sind. Derartige chirurgische Instrumente können beispielsweise zahnärztliche Bohrer oder Fräser sein, sie können auch als Shaver-Instrumente für die Arthroskopie ausgebildet sein.

Aus dem Stand der Technik ist es bekannt (siehe beispielsweise EP 1 006 898 B1), das Handstück mit einer zentrischen Bohrung oder Ausnehmung zu versehen, in welche ein Kupplungsbereich oder Ansatzbereich des chirurgischen Instruments eingesteckt wird. Dabei gelangen entsprechende Antriebskupplungen des Handstücks in Eingriff mit Kupplungsteilen des chirurgischen Instruments, so dass ein Drehantrieb realisiert wird. Das Einstecken und Verrasten des chirurgischen Instruments in dem Handstück erfolgt dadurch, dass das Handstück mit einer Verrastungsausnehmung versehen ist, mit welcher ein Rastelement des chirurgischen Instruments lösbar in Eingriff bringbar ist. Die Verrastungsausnehmung kann beispielsweise in Form einer Ringnut ausgebildet sein, es ist auch möglich, diese nur singulär vorzusehen. Das Rastelement des chirurgischen Instruments kann beispielsweise in Form eines Rasthakens, einer Rastklinke oder Ähnlichem ausgebildet sein. Beim Einstecken des chirurgischen Instruments in das Handstück kann somit eine Verrastung oder Kupplung erfolgen, die ein unbeabsichtigtes Ablösen des chirurgischen Instruments von dem Handstück verhindert. Um das chirurgische Instrument von dem Handstück zu entnehmen, ist beispielsweise eine Betätigungseinrichtung zu bedienen.

Die aus dem Stand der Technik bekannten Vorrichtungen sind vielfach kompliziert aufgebaut und entsprechend kostenintensiv und aufwendig in der Herstellung und unter operativen Einsatzbedingungen vielfach schlecht betätigbar.

Aus der US 5 871 493 A sind unterschiedlichste Ausgestaltungsvarianten chirurgischer Kupplungsvorrichtungen vorbekannt gemäß dem Oberbegriff des Anspruch 1. Bei einer Ausgestaltungsvariante ist ein die Kupplungsvorrichtung klammerartig umgreifendes Riegelelement vorbekannt, welches mittels einer Spiralfeder radial nach außen vorgespannt ist.

Der Erfindung liegt die Aufgabe zugrunde, eine chirurgische Kupplungsvorrichtung der eingangs genannten Art zu schaffen, welche bei einfachem Aufbau und einfacher, kostengünstiger Herstellbarkeit sicher und einfach bedienbar ist.

Erfindungsgemäß wird die Aufgabe durch die Merkmalskombination des Hauptanspruchs gelöst, die Unteransprüche zeigen weitere vorteilhafte Ausgestaltungen der Erfindung.

Erfindungsgemäß ist somit vorgesehen, dass das Rastelement an einem radial verschiebbaren Riegelelement befestigt ist. Dieses wird wischen einer Ruheposition und einer Freigabeposition verschoben, wobei das Riegelelement mittels eines elastischen Elements in die verriegelnde Ruheposition vorgespannt ist. Die Bedienungsperson kann somit auf einfachste Weise durch Druck auf das Betätigungselement das Riegelelement lösen, so dass das chirurgische Instrument von dem Handstück gelöst und entnommen werden kann.

Der einfache Aufbau der erfindungsgemäßen Ausgestaltung stellt somit sicher, dass auch unter schwierigsten operativen Bedingungen ein einfaches Lösen sichergestellt ist. Dieses kann insbesondere auch dann einfach erfolgen, wenn die Bedienungsperson (Operateur, Arzt) OP-Handschuhe trägt oder aufgrund der räumlichen und zeitlichen Gegebenheiten wenig Aufmerksamkeit auf den Entriegelungsvorgang richten kann.

Durch die elastische Vorspannung des Riegelelements ist es möglich, das chirurgische Instrument direkt in das Handstück einzuschieben, wodurch eine Verrastung automatisch erfolgt. Die Bedienungsperson braucht somit keine weiteren Handgriffe oder Betätigungsvorgänge auszuführen. Die erfolgreiche Einrastung lässt sich an der Stellung des Betätigungselements erkennen, so dass auch eine visuelle Überprüfung leicht und zuverlässig erfolgen kann.

Der Vorteil der erfindungsgemäßen Ausgestaltung liegt auch darin, dass das Riegelelement in einfachster Weise bewegbar ist. Hierdurch ist eine einfache und kostengünstige Grundkonstruktion möglich, die in niedrigen Herstellungskosten resultiert und besonders zuverlässig wirkt. Auch die Montage ist erfindungsgemäß sehr einfach und kostengünstig realisierbar. Da es sich bei dem chirurgischen Instrument um ein häufig auszuwechselndes Verschleißteil handelt, spielen diese Aspekte eine besonders große Rolle.

Besonders günstig ist es, wenn das Riegelelement im Wesentlichen plattenförmig ausgebildet ist. Hierdurch ist eine gute Betätigbarkeit gewährleistet, welche u.a. durch eine großflächige Kraftübertragung und eine verkantungsfreie Führung gewährleistet sein kann.

Das Riegelelement ist bevorzugterweise in einer Nut des chirurgischen Instruments angeordnet und kann auf diese Weise sicher in radialer Richtung geführt werden.

Das Betätigungselement umfaßt einen manuell betätigbaren Druckknopf, welcher somit ausreichend groß und in ergonomischer Ausgestaltung dimensioniert werden kann, um auch unter Operationsbedingungen eine sichere Handhabung zu gewährleisten. Der Druckknopf ist mit einer Druckplatte verbunden, welche radial außerhalb gegen das Riegelelement anliegt. Hierdurch wird eine gute Kraftübertragung von dem Betätigungselement (Druckknopf) auf das Riegelelement sichergestellt.

Das elastische Element ist bevorzugterweise in Form einer Blattfeder ausgebildet, welche kostengünstig herstellbar ist und eine gleichmäßige, verkantungsfreie Vorspannung gewährleistet. Ein weiterer Vorteil dieser Konstruktion liegt darin, dass die Blattfeder keine zusätzlichen Aufnahme- oder Führungselemente benötigt, sondern in einfacher Weise radial unterhalb der Entriegelungsplatte angeordnet werden kann. Durch geeignete Dimensionierung der Blattfeder ist es möglich, die Entriegelungskraft oder Auslösekraft geeignet vorzubestimmen.

Im Folgenden wird die Erfindung anhand eines Ausführungsbeispiels in Verbindung mit der Zeichnung beschrieben. Dabei zeigt:
- Fig. 1: eine axiale Teil-Schnittansicht der erfindungsgemäßen chirurgischen Kupplungsvorrichtung, und
- Fig. 2: eine Explosionsansicht der erfindungsgemäß wichtigsten Bauteile der Kupplungsvorrichtung.

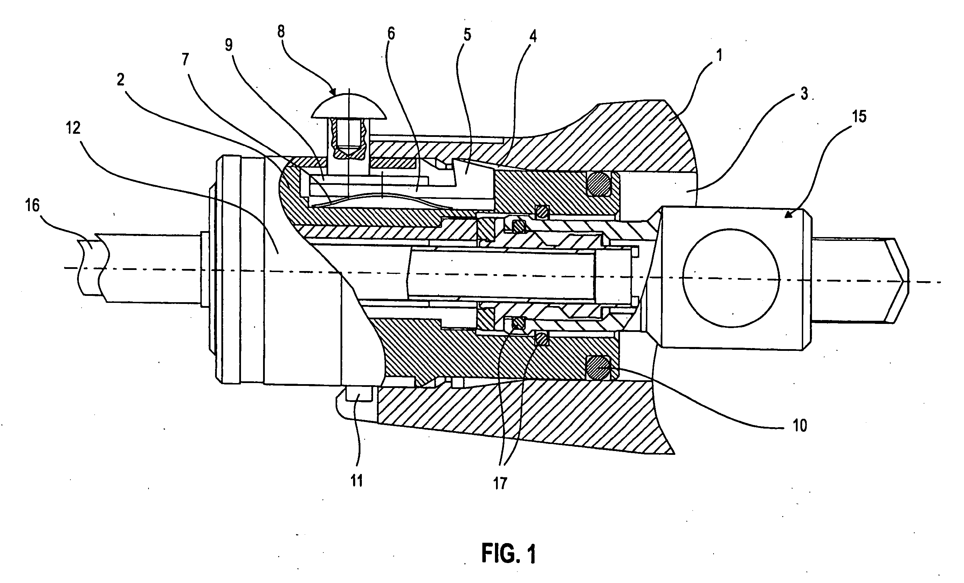

Die Fig. 1 zeigt in axialer Schnittansicht einen Teil eines Handstücks 1. Dieses bildet einen Teil eines Gehäuses eines Antriebs, um eine nachfolgend noch zu beschreibende Klinge in Drehung zu versetzen. Das Handstück 1 wird vom Operateur während der Benutzung eines chirurgischen Instrumentes gehalten.

Das Handstück 1 umfasst eine zentrische, im Wesentlichen zylindrische Ausnehmung 3, in welche ein Endbereich oder Kupplungsbereich eines chirurgischen Instruments 2 einschiebbar ist. Eine Abdichtung gegenüber dem Gehäuse des Handstücks 1 erfolgt dabei mittels eines O-Rings 10.

Zur axialen Sicherung des chirurgischen Instruments 2 weist dieses ein Riegelelement 6 mit einem Rastelement 5 auf, welches in eine kreisringförmige Verrastungsausnehmung 4 einrastbar ist, welche an der Wandung der Ausnehmung 3 ausgebildet ist.

Die Fig. 1 zeigt eine Abdeckkappe 12, welche auf den Endbereich des chirurgischen Instruments 2 aufgesteckt ist und eine Ausnehmung aufweist, durch welche ein Druckknopf 8 gehalten und positioniert ist, welcher mit einer Druckplatte 9 verbunden ist, welche gegen ein plattenförmiges Riegelelement 6 anliegt. Durch Druck auf den Druckknopf 8 in radialer Richtung wird das Riegelelement 6 gegen die Kraft eines elastischen Elements 7 (Blattfeder) verschoben, so dass sich das Rastelement 5 aus der Verrastungsausnehmung 4 löst und in eine Freigabeposition bringbar ist.

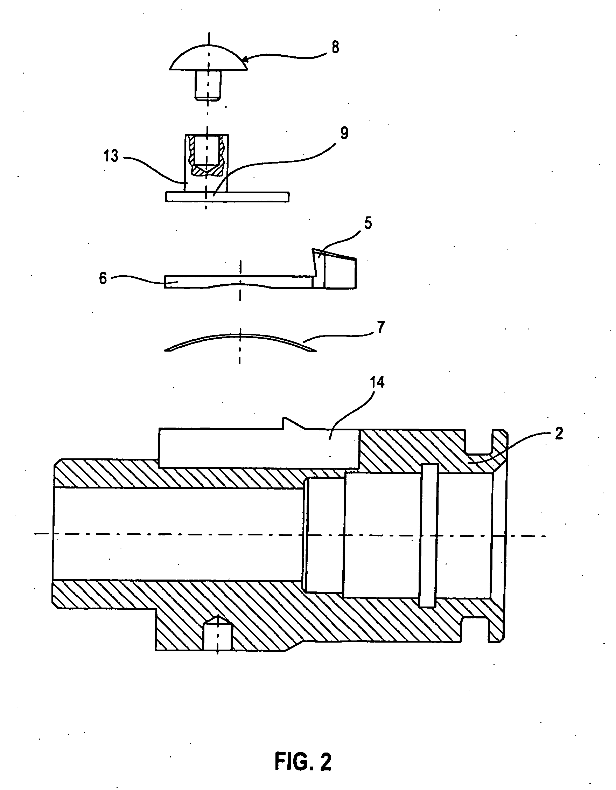

Der Aufbau der einzelnen Elemente ist aus der Explosionsansicht der Fig. 2 ersichtlich.

Das Rastelement 5 ist keilförmig ausgebildet bzw. abgeschrägt, so dass dieses beim Einschieben des chirurgischen Instruments 2 in das Handstück 1 durch die Innenwandung der Ausnehmung 3 radial nach Innen gegen die Kraft der Blattfeder 7 gedrückt wird, um ein automatisches Verrasten zu bewirken.

Wie insbesondere aus der Fig. 2 ersichtlich ist, ist mit der Druckplatte 9 ein im Wesentlichen zylindrischer Ansatz 13 verbunden, der ein Innengewinde aufweist, in welches ein Gewinde des Druckknopfes 8 einschraubbar ist. Hierdurch kann der Druckknopf 8 montiert werden.

Wie die Fig. 1 zeigt, sichert die Abdeckkappe sowohl das Riegelelement 6, welches in einer Nut 14 geführt ist, als auch die Blattfeder 7, welche radial innerhalb des Riegelelements 6 gegen dieses und gegen den Nutgrund der Nut 14 anliegt. Weiterhin sichert die Abdeckkappe 12 die Druckplatte 9 und hält diese in ihrer Position.

Durch geeignete Dimensionierung und Formgestaltung der Blattfeder 7 wird erreicht, dass ein Druck auf den Druckknopf 8 das Riegelelement 6 im Wesentlichen in Radialrichtung verschiebt, nicht jedoch verkantet oder verschwenkt, um das Rastelement 5 aus der in Fig. 1 gezeigten Ruheposition in eine Freigabeposition zu verschieben.

Die Fig. 1 zeigt weiterhin einen Teil eines Antriebs 15 für eine Klinge 16 (Shaver-Blade). Sowohl der Antrieb 15 als auch die Klinge 16 sind aus dem Stand der Technik vorbekannt, so dass auf diesen verwiesen werden kann (siehe beispielsweise DE 696 31 340 T2). Eine Abdichtung des Antriebs 15 (Antriebswelle) gegenüber dem chirurgischen Instrument 2 (Gehäuse des Shaver-Blades) erfolgt über O-Ringe 17. Die Kupplung des Antriebs 15 mit der Klinge 16 sowie die diesbezüglichen weiteren Ausgestaltungen sind aus dem Stand der Technik (siehe oben) vorbekannt, so dass auf eine detaillierte Beschreibung an dieser Stelle verzichtet werden kann. Gleiches gilt für einen Positionierungsstift 11, welcher eine Drehsicherung bildet.

### Bezugszeichenliste

- 1: Handstück
- 2: Chirurgisches Instrument (Gehäuse des Shaver-Blades)
- 3: Ausnehmung
- 4: Verrastungsausnehmung
- 5: Rastelement
- 6: Riegelelement
- 7: Elastisches Element (Blattfeder)
- 8: Druckknopf
- 9: Druckplatte
- 10: O-Ring
- 11: Positionierungsstift
- 12: Abdeckkappe
- 13: Ansatz
- 14: Nut
- 15: Antrieb des Shaver-Blades
- 16: Klinge des Shaver-Blades

## Patentansprüche

1. Chirurgische Kupplungsvorrichtung zur lösbaren Verbindung eines Handstücks (1) mit einem chirurgischen Instrument (2), wobei das Handstück (1) mit einer Ausnehmung (3) versehen ist, in welche ein Kupplungsbereich des Instruments (2) lösbar einschiebbar ist,
wobei an einer innenliegenden Wandung der Ausnehmung (3) eine Verrastungsausnehmung (4) ausgebildet ist,
mit welcher ein Rastelement (5) lösbar in Eingriff bringbar ist, welches an dem Instrument (2) gelagert ist,
**dadurch gekennzeichnet,**
**dass** das Rastelement (5) an einem sich im Wesentlichen in Axialrichtung erstreckenden, radial zwischen einer verrastenden Ruheposition und einer Freigabeposition verschiebbar an dem Instrument (2) gelagerten, im Wesentlichen plattenförmigen Riegelelement (6) ausgebildet ist, welches mittels zumindest eines elastischen Elements (7) in Form einer Blattfeder radial nach außen in die Ruheposition vorgespannt ist, wobei das Riegelelement (6) mittels eines Betätigungselements gegen die Vorspannung des elastischen Elements (7) in eine Freigabeposition bewegbar ist,
wobei das Betätigungselement einen manuell betätigbaren Druckknopf (8) umfasst, welcher mit einer Druckplatte (9) verbunden ist, welche radial außerhalb gegen das Riegelelement (6) anliegt.

2. Vorrichtung nach Anspruch 1, **dadurch gekennzeichnet, dass** das Riegelelement (6) in einer Nut (14) des chirurgischen Instruments (2) angeordnet ist.

3. Vorrichtung nach einem der Ansprüche 1 oder 2, **dadurch gekennzeichnet, dass** das Riegelelement (6), das elastische Element (7) sowie die Druckplatte (9) mittels einer Abdeckkappe (12) radial nach außen in der Nut (14) gesichert sind.

## Claims

1. A surgical coupling device for releasably connecting a handpiece (1) to a surgical instrument (2), the handpiece (1) being provided with a recess (3) into which a coupling portion of the instrument (2) can be detachably inserted,
a locking recess (4) being formed at an interior wall of the recess (3),
with which a locking element (5) can be brought into detachable engagement, said locking element (5) being supported on the instrument (2),
**characterized in that**
the locking element (5) is formed on a plate-shaped latch element (6) which extends substantially in axial direction and which is radially displaceably supported on the instrument (2) between a locked inoperative position and a release position, which is biased by means of at least one elastic element (7) in the form of a plate spring radially outwards into the inoperative position, the latch element (6) being movable by means of an actuating element against the bias of the elastic element (7) into a release position,
wherein the actuating element comprises a manually operable pushbutton (8) which is connected to a pressure plate (9) which abuts radially outside against the latch element (6).

2. The device according to claim 1, **characterized in that** the latch element (6) is arranged in a groove (14) of the surgical instrument (2).

3. The device according to any one of claims 1 or 2, **characterized in that** the latch element (6), the elastic element (7) and the pressure plate (9) are secured by means of a covering cap (12) radially outwards in the groove (14).

## Revendications

1. Dispositif de couplage chirurgical pour l'assemblage amovible d'une pièce à main (1) à un instrument (2) chirurgical, ladite pièce à main (1) étant munie d'un évidement (3), dans lequel peut être insérée de manière amovible une zone de couplage de l'instrument (2),
un évidement de blocage (4) étant réalisé contre une paroi intérieure de l'évidement (3),
dans lequel un élément de blocage (5), qui est monté sur l'instrument (2), peut être amené en prise amovible,
**caractérisé en ce que**
l'élément de blocage (5) est réalisé sur un élément de verrouillage (6) sensiblement en forme de plaque, orienté sensiblement dans la direction axiale, qui est monté sur l'instrument (2) de manière radialement mobile entre une position de repos bloquante et une position de déblocage, et qui, au moyen d'au moins un élément (7) élastique en forme de ressort à lame est précontraint radialement vers l'extérieur dans la position de repos, ledit élément de verrouillage (6) étant apte à être déplacé au moyen d'un élément de manoeuvre dans une position de déblocage à l'encontre de la précontrainte de l'élément (7) élastique,
l'élément de manoeuvre comportant un bouton-poussoir (8) actionnable manuellement, qui est relié à une plaque de pression (9), qui est en appui radialement à l'extérieur contre l'élément de verrouillage (6).

2. Dispositif selon la revendication 1, **caractérisé en ce que** l'élément de verrouillage (6) est disposé dans une rainure (14) de l'instrument (2) chirurgical.

3. Dispositif selon la revendication 1 ou 2, **caractérisé en ce que** l'élément de verrouillage (6), l'élément (7) élastique, ainsi que la plaque de pression (9) sont bloqués dans la rainure (14) radialement vers l'extérieur au moyen d'un cache de fermeture (12).
